# EUROPÄISCHE PATENTANMELDUNG

(11) **EP 3 097 981 A1**
(43) Veröffentlichungstag der Anmeldung: **30.11.2016**
(21) Anmeldenummer: 15169339.7
(22) Anmeldetag: 27.05.2015
(51) Int. Cl.: B05B 1/26, A61M 11/06, A61M 15/00, B65D 83/14

(54) **SPRÜHKOPF FÜR EINE SPRÜHDOSE, VERFAHREN ZU DESSEN HERSTELLUNG UND ZERSTÄUBUNGSVERFAHREN**

(71) Anmelder: Taren-Salt UG, 14089 Berlin (DE)
(72) Erfinder: Rentsch, Rüdiger, 14089 Berlin (DE)
(74) Vertreter: Ettmayr, Andreas

(57) **Zusammenfassung**

Das Ventil des mit dem Sprühkopf (1) verbundenen Sprühbehälters (2) ähnelt den Ventilen herkömmlicher Sprühdosen. Um die Handhabung zu erleichtern und Verkannten beim Zusammendrücken zu vermeiden, ist der Sprühbehälter (2) in einer mit dem Sprühkopf (1) verbundenen Adapterhülle (11) geführt. Eingesetzt in die Sprühkopfkulisse (13) ist der Düsenkörper (14). Mittels Laserbearbeitung sind in den Düsenkörper (14) zwei im Winkel α zueinander geneigte Austrittskanäle (15a, 15b) gebohrt. Die Oberfläche des Düsenkörpers (14) auf dessen Austrittsseite ist so angeschrägt, dass die von den Rändern der Austrittsöffnungen (16a, 16b) aufgespannten Austrittsquerschnittsflächen ebenfalls zueinander geneigt sind. Den Winkel α, Querschnitt und Länge der Sprühkanäle (15a, 15b) kann der Fachmann variieren, um eine gewünschte Tröpfchengrößenverteilung im beim Sprühen ausgestoßenen Aerosol zu erhalten.

## Beschreibung

Die vorliegende Erfindung betrifft einen Sprühkopf für eine Sprühdose, ein Verfahren zu dessen Herstellung und ein Zerstäubungsverfahren.

Sprühdosen, oft auch als Spraydosen bezeichnet, sind seit Jahrzehnten in den unterschiedlichsten Anwendungsgebieten bekannt. Sie weisen einen Sprühbehälter auf, in welchem sich ein unter Druck stehendes Treibmittel und das zu versprühende flüssige Sprühgut befindet, sowie einen relativ zum Sprühbehälter beweglich gelagerten Sprühkopf mit mindestens einer Austrittsdüse, aus welcher das Sprühgut austreten kann, wenn durch Bewegen des Sprühkopfes ein Ventil betätigt wird. Je nach Anwendungsgebiet sind dem Fachmann verschiedenste technische Ausführungsformen von Sprühdosen und deren Bestandteilen geläufig.

Die Einheit aus Sprühdose, Treibmittel und Sprühgut wird gemeinhin als Spray bezeichnet. Im medizinischen bzw. pharmazeutischen Bereich finden Sprays insbesondere zur Behandlung von Atemwegserkrankungen Verwendung, beispielsweise von leichten Erkältungserkrankungen mit Beschwerden der oberen Luftwege, aber auch von asthmatischen Beschwerden und anderen Erkrankungen der unteren Luftwege. Zudem können auch Wirkstoffe mittels Sprays verabreicht werden, welche nicht zur lokalen Wirkung in den Luftwegen bestimmt sind, sondern über die Luftwege nur aufgenommen und dann anderweitig im Patientenkörper wirksam werden sollen, beispielsweise Adrenalin zur Behandlung von Schockzuständen.

Die Einsatzmöglichkeiten herkömmlicher Sprühdosen sind begrenzt. Im medizinischen Bereich besteht eine der Grenzen darin, dass sich das Sprühgut für manche Anwendungen mittels herkömmlicher Sprühdosen nicht fein genug zerstäuben lässt, d.h. insbesondere die Erzeugung besonders feiner, lungengängiger Tröpfchen nicht in ausreichendem Maße möglich ist. Einschränkungen beruhen zum einen darauf, dass Behälterdrücke in Sprühbehältern aus Sicherheits- und Kostengründen in der Regel 15 bar kaum überschreiten, zum anderen darauf, dass die Austrittskanäle für das Sprühgut in Sprühkopfdüsen aus fertigungstechnischen Gründen nicht beliebig fein zu wirtschaftlichen Konditionen herstellbar sind.

Ferner eignen sich medizinische Sprays in der Regel nur zur Verabreichung einzelner kurzer Sprühstöße, einer längeren kontinuierlichen Sprühdauer sind durch den Vorrat an Sprühgut und Treibmittel Grenzen gesetzt, zumal die Dosierbarkeit herkömmlicher Sprays eher grob ist, d.h. die Verringerung des austretenden Stroms an Sprühgut zugunsten einer längeren Sprühdauer für den Benutzer in der Regel nicht umsetzbar ist.

Eine Alternative zu medizinischen Sprays stellen technisch aufwendigere Inhalatoren dar, welche mit anderen Energiequellen als komprimiertem Treibmittel arbeiten, beispielsweise elektromechanisch oder mittels Ultraschallzerstäubung. Aufgrund des technischen Aufwands und der damit verbundenen Kosten ist das Anwendungsspektrum derartiger Inhalatoren ebenfalls begrenzt. Insbesondere lassen sie sich nicht wie ein Spray einfach einstecken um bei Bedarf, beispielsweise während sportlicher Betätigung, auf Reisen, oder im normalen Alltagsgeschehen, sofort zur Verfügung zu stehen.

Neben Inhalatoren und herkömmlichen Sprühdosen sind auch Zerstäuberpumpen bekannt, die mittels eines hochgespannten Federsystems im Sprühgut sehr hohe Drücke, teilweise über 200 bar erreichen können. In Kombination mit mikrostrukturierten Düsenkörpern können hiermit sehr feine, lungengängige Tröpfchen erzeugt werden, allerdings nur während kurzer Sprühstöße von maximal etwa 1,5 Sekunden Dauer. Entsprechende Düsenkörper sind zudem aufwendig in der Herstellung. Dabei werden feine Kanäle mittels lithographischer Verfahren aus einem Siliziumdüsenkörper herausgeätzt. Derartige Zerstäuberpumpen und zugehörige Sprühköpfe sind beispielsweise aus EP 1493492 A1 und EP 1386670 A2 bekannt.

Angesichts der Einschränkungen denen der Einsatz herkömmlicher Sprühdosen, Inhalatoren und Zerstäuberpumpen unterliegt, liegt der vorliegenden Erfindung die Aufgabe zugrunde, eine Vorrichtung und eine Methode bereitzustellen, mit möglichst geringem Kostenaufwand geringe Tröpfchendurchmesser zu erzielen, insbesondere zum Verabreichen von medizinischen Wirkstoffen in die Atemwege eines Patienten.

Gemäß einem Aspekt der vorliegenden Erfindung wird diese Aufgabe durch einen mit Behälterverbindungsmitteln zum Verbinden mit einem Sprühbehälter ausgestatteten Sprühkopf gelöst, der mindestens zwei Austrittskanäle aufweist, welche so angeordnet sind, dass aus Austrittsöffnungen der Austrittskanäle austretende Sprühstrahle an einem von den Austrittsöffungen beabstandeten Aufprallort zentral aufeinander treffen. Zentral bedeutet dabei, dass der überwiegende Teil des aus dem einen Austrittskanal als Sprühstrahl austretenden Sprühguts auf den überwiegenden Teil des aus dem anderen Austrittskanal als Sprühstrahl austretenden Sprühguts auftrifft. Eine feine Zerstäubung wird hier insbesondere dadurch erreicht, dass unter Druck ausgestoßenes Sprühgut auf unter Druck ausgestoßenes Sprühgut prallt. In der Regel genügt es und ist in Hinblick auf eine kostengünstige Fertigung auch bevorzugt, zwei Austrittskanäle vorzusehen. Jedoch kann der Sprühkopf vorteilhaft auch mit drei oder mehr Austrittskanälen versehen sein.

Vorteilhaft umsetzbar ist eine solche Anordnung beispielsweise, indem zwischen jeweils zweien der Austrittskanäle der Abstand deren Eintrittsöffnungen größer ist als der Abstand deren Austrittsöffnungen. Der Abstand ist dabei als Abstand zwischen den Flächenschwerpunkten der Flächen definiert, welche jeweils von den Rändern der Eintrittsöffnungen bzw. Austrittsöffnungen aufgespannt werden.

Vorzugsweise weisen die Austrittskanäle jeweils eine Mittelachse aufweisen, welche zur jeweiligen Mittelachse eines jeweils anderen der Austrittskanäle in einem Winkel zwischen 30 und 120 Grad, besonders bevorzugt zwischen 45 und 90 Grad steht. Um einen maximalen zur zerstäubung nutzbaren Energieeintrag beim Aufprall der Sprühstrahle aufeinander zu erzielen, kann der Winkel jedoch 120 Grad auch überschreiten und bis zu 180 Grad (entsprechend direkt aufeinander gerichteter Sprühstrahle) betragen.

Gemäß einer besonders bevorzugten Ausführungsform sind die vom Rand der jeweiligen Austrittsöffnungen aufgespannten Austrittsquerschnittsflächen zueinander geneigt, d.h. eine auf der einen Austrittsquerschnittsfläche stehende Orthogonale strebt einer auf der anderen Austrittsquerschnittsflächen stehenden Orthogonale zu.

Vorzugsweise weisen die Austrittskanäle jeweils einen kleinsten Durchmesser zwischen 5 und 50 µm, besonders bevorzugt zwischen 5 und 15 µm auf.

Gemäß einer weiteren bevorzugten Ausführungsform weisen die Austrittskanäle jeweils eine Länge von 50 bis 200 µm auf. Die Länge ist dabei als Abstand zwischen den Flächenschwerpunkten der von den Rändern der Eintrittsöffnung und Austrittsöffnung eines Austrittskanals aufgespannten Flächen definiert.

Vorzugsweise beträgt das jeweils als Quotient aus Länge eines Austrittskanals und geringstem Durchmesser des Austrittskanals gebildete Aspektverhältnis zwischen 3 und 30, besonders bevorzugt zwischen 10 und 25.

Vorzugsweise sind die Austrittskanäle in einem gemeinsamen Düsenkörper angeordnet, der als eigenes Bauteil oder integral mit dem restlichen Sprühkopf ausgebildet sein kann. Dies ermöglicht eine höhere Fertigungsgenauigkeit.

Vorteilhafterweise kann der Düsenkörper aus einem Kunststoff, beispielsweise Polyethylen, gefertigt sein, was dazu beiträgt, die Fertigungskosten gering zu halten. Jedoch können auch andere, beispielsweise metallische, Werkstoffe verwendet werden.

Ebenfalls kostengünstig kann der Düsenkörper als Spritzgussteil ausgeführt sein.

Vorzugsweise weist der Sprühkopf einen gemeinsamen Zuführungskanal zum Zuführen von Sprühgut zu Eingangsöffnungen der Austrittskanäle auf. Je nachdem, wie breit oder schmal die Auffächerung des beim Sprühvorgang erzeugten Aerosol-Strahls erwünscht ist, können die Austrittskanäle an ihren Austrittsöffnungen vorteilhafterweise in einen weiteren oder engeren Sprühkanal münden.

Gemäß einer besonders vorteilhaften Ausführungsform weist der Sprühkopf Adapterverbindungsmittel für einen Patientenluftwegeadapter auf, wobei der Sprühkopf mittels der Adapterverbindungsmittel werkzeugfrei, beispielsweise mittels einer Steck- oder Schraubverbindung, so mit dem Patientenluftwegeadapter verbindbar ist, dass aus den Austrittsöffnungen ausgetretenes Sprühgut in den Patientenluftwegeadapter eintreten kann. Ein solcher Patientenluftwegeadapter als in eine Patientennase einführbarer Nasenadapter, als Mundstück, oder als einen Patientenmund und/oder eine Patientennase abdeckender Maskenadapter ausgebildet sein.

Ebenfalls vorteilhaft kann der Sprühkopf mit einem Patientenluftwegeadapter integral geformt oder verbunden sein, der wiederum als in eine Patientennase einführbarer Nasenadapter, als einen Patientenmund und/oder eine Patientennase abdeckender Maskenadapter oder als mit einem Patientenmund umschließbarer Adapter ausgebildet ist.

Vorteilhafterweise können die Patientenluftwegeadapter aus einem geeigneten Kunststoff wie etwa PE oder PP, beispielsweise als Spritzgussteil, ausgeführt sein.

Gemäß einer weiteren vorteilhaften Ausführungsform kann der Sprühkopf mit einer Adapterhülle zum Einführen eines Sprühbehälters verbunden oder integral ausgebildet sein, wobei über die Behälterverbindungsmittel eine werkzeugfrei schließbare und werkzeugfrei lösbare Verbindung mit dem Sprühbehälter herstellbar ist. So lässt sich der Sprühkopf mit geeigneten Einweg-Sprühbehältern nutzen und resourcenschonend mehrfach verwenden.

Besonders bevorzugt werden die Austrittskanäle mittels Laserbearbeitung hergestellt. Eingesetzt werden dabei bevorzugt Pico- oder Femto-Laser, wobei sich bei Einsatz eines Femto-Lasers eine höhere Fertigungsgenauigkeit bei geringerer Materialerwärmung und glatterer Oberfläche erzielen lässt. Der Laser wird dabei in Richtung des gewünschten Kanalverlaufs wie ein Bohrer eingesetzt. Durch den in oberflächennahen Schichten höheren Energieeintrag ergibt sich eine konische Kanalform. Je nachdem, ob die Bearbeitung von der Seite der späteren Eintrittsöffnung oder der Seite der späteren Austrittsöffnung her erfolgt, ist die engere Seite des Konus an der Austrittsöffnung bzw. Eintrittsöffnung. Denkbar ist auch eine Bearbeitung von beiden Seiten, was jedoch eine besonders hohe Präzision bei der Laserausrichtung erfordert.

Demgemäß betrifft die vorliegende Erfindung gemäß einem Aspekt auch ein Verfahren zum Herstellen eines Düsenkörpers für einen Sprühkopf, wobei mittels Laserbearbeitung Düsenkörpermaterial zum Erzeugen eines Austrittskanals vom Düsenkörper abgetragen wird. Gemäß einer bevorzugten Ausführungsform wird, wie oben angedeutet, ein mittels Spritzguss gefertigter Düsenkörper, vorzugsweise aus Kunststoff, beispielsweise PE, zur Bearbeitung vorgelegt.

Vorzugsweise werden die mindestens zwei Austrittskanäle in demselben Düsenkörper erzeugt, da dies die mögliche Fertigungspräzision gegenüber der ebenfalls möglichen Produktion eines eigenen (bei der späteren Montage auf den jeweils anderen Düsenkörper auszurichtenden) Düsenkörpers für jeden Austrittskanal erhöht.

Vorzugsweise bleibt der Düsenkörper während dem Erzeugen beider Austrittskanäle ortsfest, d.h. verschiedene Einstrahlungswinkel des Lasers gegenüber dem Düsenkörper werden durch Verstellen der Laseroptik bzw. -lichtquelle anstelle einer Lageveränderung des Werkstücks realisiert.

Ebenfalls betrifft die Erfindung gemäß einem Aspekt ein Verfahren zum Herstellen eines Sprühkopfes der oben dargestellten Art, wobei mindestens ein wie oben hergestellter Düsenkörper in eine Sprühkopfkulisse eingesetzt wird. Die Sprühkopfkulisse kann vorteilhafterweise wiederum aus Kunststoff gefertigt sein. Alternativ kann der Düsenkörper jedoch auch mit weiteren Teilen des Sprühkopfes oder dem gesamten Sprühkopf integral bzw. einstückig ausgebildet sein. Das bedeutet, vorteilhafterweise können die Austrittskanäle auch direkt in den Sprühkopf oder Teile hiervon eingearbeitet werden, ohne dass es eines separaten Düsenkörpers überhaupt bedarf.

Gemäß einem weiteren Aspekt betrifft die Erfindung eine Sprühdose, welche einen Sprühkopf der obigen Art und den Sprühbehälter aufweist. Vorzugsweise übersteigt der Druck im Sprühbehälter 30 bar nicht, bevorzugt übersteigt er 15 bar, besonders bevorzugt 10 bar nicht.

Auch im Bereich von Behälterdrücken von 3 bis 10 bar erreicht die erfindungsgemäße Sprühdose noch eine wesentlich feinere Tröpfchengrößen als herkömmliche Sprühdosen mit Treibmittel.

Gemäß einem weiteren Aspekt betrifft die Erfindung ein Verfahren zur Herstellung einer Sprühdose der obigen Art, welches das Herstellen eines Sprühkopfes der obigen Art und das Verbinden des hergestellten Sprühkopfes mit einem Sprühbehälter umfasst.

Für den besonders bevorzugten Anwendungsbereich der erfindungsgemäßen Sprühdose ist der Sprühbehälter mit zumindest einem medizinischen Wirkstoff, vorzugsweise Sole, und einem Treibmittel befüllt. Insbesondere bei der Verwendung von Sole kommt der erfindungsgemäße Vorteil zum Tragen, dass sich eine breite Tröpfchengrößenverteilung erzielen lässt. Sole kann so vorteilhafterweise an die oberen und unteren Luftwege gleichermaßen verabreicht werden. Insbesondere Sole eignet sich auch deshalb für eine gleichzeitige Verabreichung an die oberen und unteren Luftwege, da es sich hierbei um einen weitestgehend nebenwirkungsfreien Stoff handelt. Wenn bei einer der Behandlung eines Patienten vorausgehenden Diagnose oder Selbstdiagnose nicht eindeutig klar ist, in welchem Bereich der Luftwege die Solegabe benötigt wird, kann mit Tröpfchen unterschiedlicher Größe ein vorsorglich auf unterschiedliche Bereiche der Luftwege zugleich einwirkender Therapieansatz verfolgt werden.

Entsprechend betrifft die vorliegende Erfindung gemäß einem weiteren Aspekt auch ein Zerstäubungsverfahren, bei welchem Sprühgut mittels Behälterdrucks in Form mindestens zweier aufeinander prallender Sprühstrahle aus einem Sprühkopf gedrückt wird.

Insbesondere bei Einsatz eines Sprühkopfes bzw. einer Sprühdose der obigen Art gestattet das erfindungsgemäße Zerstäubungsverfahren die Erzeugung von Sprühtröpfchen mit einer Tröpfchengrößenverteilung, bei welcher der Sauterdurchmesser zwischen 5 und 25 µm beträgt. Bevorzugt liegt der Sauterdurchmesser der Tröpfchen zwischen 5 und 15 µm, was eine gute Lungengängigkeit bedeutet.

Vorzugsweise beträgt der Volumenstrom an Sprühgut zwischen 0,1 und 0,9 ml pro Minute.

Tröpfchengrößenverteilung und Volumenstrom lassen sich durch geeignete Wahl der o.a. Parameter Durchmesser bzw. Querschnittsfläche der Austrittsöffnungen der Austrittskanäle, Länge der Austrittskanäle, Winkel der Austrittskanäle bzw. deren Mittelachsen zueinander, Neigungswinkel der Austrittsöffnungen zueinander und Behälterdruck wunschgemäß einstellen.

Die Erfindung wird nachfolgend in beispielhafter Weise anhand der beigefügten schematischen Zeichnung näher erläutert. Die Zeichnungen sind nicht maßstabsgetreu; insbesondere entsprechen Verhältnisse der einzelnen Abmessungen zueinander aus Gründen der Anschaulichkeit teilweise nicht den Abmessungsverhältnissen in tatsächlichen technischen Umsetzungen. Es werden mehrere bevorzugte Ausführungsbeispiele beschrieben, auf welche die Erfindung jedoch nicht beschränkt ist. Grundsätzlich kann jede im Rahmen der vorliegenden Anmeldung beschriebene bzw. angedeutete Variante der Erfindung besonders vorteilhaft sein, je nach wirtschaftlichen, technischen und ggf. medizinischen Bedingungen im Einzelfall. Soweit nichts gegenteiliges dargelegt ist, bzw. soweit grundsätzlich technisch realisierbar, sind einzelne Merkmale der beschriebenen Ausführungsformen austauschbar oder miteinander sowie mit per se aus dem Stand der Technik bekannten Merkmalen kombinierbar.

Es zeigt
- Fig. 1: die Querschnittsdarstellung einer erfindungsgemäßen Sprühdose mit einem erfindungsgemäßen, mit einem Nasenadapter integral geformten Sprühkopf und einem Sprühbehälter,
- Fig. 2: den Düsenkörper des Sprühkopfes aus Fig. 1, ebenfalls in Querschnittsdarstellung,
- Fig. 3: die Querschnittsdarstellung eines erfindungsgemäßen, mit einem Mundstück integral geformten Sprühkopfs mit einem Düsenkörper wie dem in Fig. 2 dargestellten, und
- Fig. 4: einen erfindungsgemäßen Sprühkopf, wiederum in Querschnittsdarstellung, mit aufgeschraubter Mund-/Nasenmaske und einem Düsenkörper wie dem in Fig. 2 dargestellten.

Einander entsprechende Elemente sind in den Zeichnungsfiguren jeweils mit den gleichen Bezugszeichen bezeichnet.

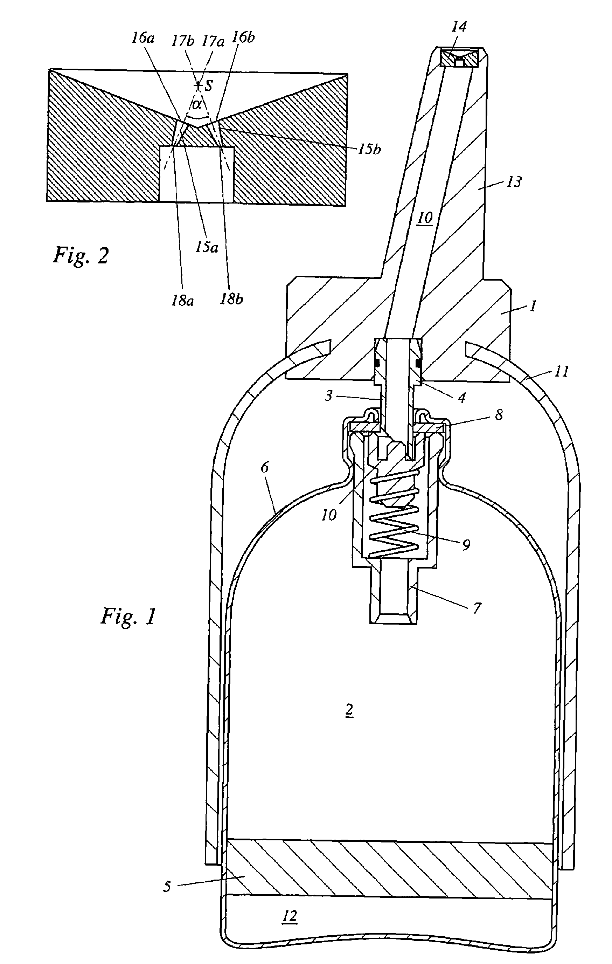

Der wiederverwendbare Sprühkopf 1 in Fig. 1 ist auf den Kopf 4 des Austrittsrohrs 3 des Sprühbehälters 2 aufgesetzt und von diesem werkstofffrei abziehbar, um den Austausch des Sprühbehälters 2 zu ermöglichen. Der Sprühbehälter 2 ist als Zweikammerbehälter ausgeführt, in welchem ein verschieblicher Kolben 5 das im unteren Bereich 12 des Sprühbehälters 2 vorhandene komprimierte Treibmittel vom Sprühgut, beispielsweise Sole, trennt. Alternativ lässt sich die Erfindung auch mit einem Einkammerbehälter als Sprühbehälter 2 ausführen, bei welchem Treibmittel und Sprühgut vermischt sind. Als Treibmittel eignen sich die aus herkömmlichen Sprays bekannten Treibmittel. Die Behälterwand 6 des Sprühbehälters 2 ist aus herkömmlichem Behältermaterial wie beispielsweise Aluminium oder Weißblech gefertigt.

Das Ventil des Sprühbehälters 2 ähnelt den Ventilen herkömmlicher Sprühdosen und weist ein Ventilgehäuse 7 auf, welches über den aus einem Elasten wie Kautschuk- oder Silikongummi bestehenden Dichtring 8 abgedichtet ist. Die ins Ventilgehäuse eingesetzte Feder 9 drückt die Verschlusskapsel 10 gegen den Dichtring 8. Durch Zusammendrücken von Sprühkopf 1 und und Sprühbehälter 2 relativ zu einander schiebt das unten angeschrägte Austrittsrohr 3 die Verschlusskapsel nach unten, so dass Sprühgut durch Ventilgehäuse 7 und Austrittsrohr 3 in den Zuführungskanal 10 des Sprühkopfs 1 eintreten kann. Um die Handhabung zu erleichtern und Verkannten beim Zusammendrücken zu vermeiden, ist der Sprühbehälter 2 in einer mit dem Sprühkopf 1 fest verbundenen Adapterhülle 11 geführt.

Der Sprühkopfkulisse 13 besteht aus thermoplastischem oder duroplastischen Kunststoff, z.B. Polyethylen, Polypropylen oder Polycarbonat, und ist als in ein Nasenloch einführbarer Nasenadapter ausgeformt. Eingesetzt in die Sprühkopfkulisse 13 ist der Düsenkörper 14, welcher in Fig. 2 vergrößert dargestellt ist.

Mittels Laserbearbeitung sind in den zylindrischen Kunststoff- oder Metall-Düsenkörper 14 zwei Austrittskanäle 15a, 15b gebohrt. Durch den Energieeintrag von der Seite der Austrittsöffnungen 16a, 16b her sind die Austrittskanäle 15a, 15b konisch. Der Winkel α zwischen den sich im Punkt S schneidenden Mittelachsen 17a, 17b der Austrittskanäle 15a, 15b beträgt etwa 40°. Die Oberfläche des Düsenkörpers 14 auf dessen Austrittsseite ist so angeschrägt, dass die von den Rändern der Austrittsöffnungen 16a, 16b aufgespannten Austrittsquerschnittsflächen zueinander geneigt sind. Vorzugsweise sind die Austrittsquerschnittsflächen dabei orthogonal zu den Mittelachsen 17a, 17b der Austrittskanäle 15a, 15b. Die Anschrägung kann als zwei einander schneidende Ebenen oder als konische Vertiefung ausgeführt sein. Das Aspektverhältnis zwischen der Länge der Austrittskanäle 15a, 15b (entlang deren Mittelachsen 17a, 17b) und deren geringstem Durchmesser an den Eintrittsöffnungen 18a, 18b beträgt 4:1.

Den Winkel α, Querschnitt und Länge der Sprühkanäle 15a, 15b kann der Fachmann variieren, um eine gewünschte Tröpfchengrößenverteilung im beim Sprühen ausgestoßenen Aerosol zu erhalten.

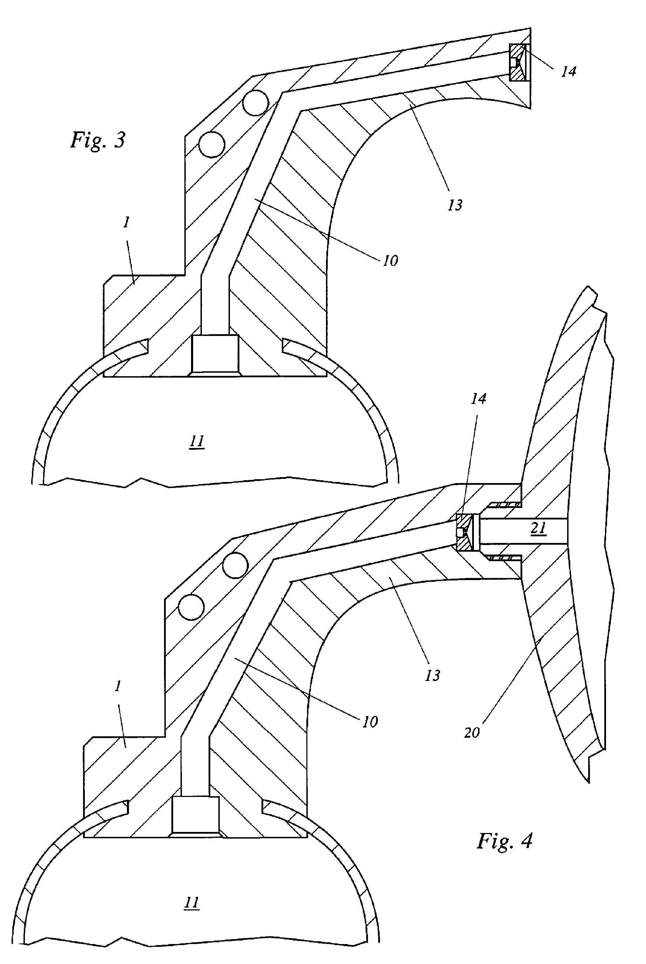

Die Sprühköpfe 1 in Fig. 3 und Fig. 4 sind ähnlich aufgebaut wie in Fig. 1 jedoch ohne den Sprühbehälter 2 abgebildet. Die Adapterhülle 11 ist jeweils nur teilweise dargestellt.

In Fig. 3 ist die Sprühkopfkulisse 13 des Sprühkopfs 1 als von den Lippen eines Patienten umschließbares Mundstück ausgeführt. Nahe an dessen Ende ist der Düsenkörper 14 eingesetzt.

In Fig. 4 weist die Sprühkopfkulisse 13 ein Gewinde 20 auf, über welches verschiedene Patientenluftwegeadapter 19 anschraubbar sind. Unterbrochen dargestellt ist ein als Mund-Nasen-Maske ausgebildeter Patientenluftwegeadapter 19, welcher aus einem festen oder vorzugsweise flexiblen Kunststoff, wie beispielsweise einem Silikonkautschuk, hergestellt sein kann, um Nase und Mund eines Patienten sicher zu umschließen. Ist die Maske selbst flexibel ausgeführt, empfiehlt sich, das Gegenstück zum Gewinde 20 der Sprühkopfkulisse dennoch aus einem festen Kunststoff auszuführen, d.h. den Patientenluftwegeadapter 19 aus zweierlei entsprechend geeigneten Materialien zusammenzufügen.

In der dargestellten Variante ist das Gewinde 20 als Innengewinde ausgeführt, so dass die Sprühkopfkulisse 13 als Mundstück dienen kann, wenn der Patientenluftwegeadapter 19 nicht aufgeschraubt ist.

Um einen breit aufgefächerten Sprühstrahl zu erhalten, empfiehlt es sich, den Sprühkanal 21, in welchen die Austrittsöffnungen 16a, 16b der Austrittskanäle 15a, 15b münden, möglichst kurz auszführen. Ein längerer Sprühkanal 21 sorgt für einen weniger aufgefächerten Sprühstrahl, wobei sich allerdings eine größere Menge Sprühgut an der Wand des Sprühkanals niederschlägt.

Beispiele erzielbarer Tröpfchengrößen und Volumenströme: Für einen Winkel von 80° zwischen den Mittelachsen von Austrittskanälen mit einem kleinsten Durchmesser von 10 µm bei 200 µm Kanallänge (entsprechend einem Aspektverhältnis von 20) wurden bei Sole mit einem Salzgehalt von 0,9 Massenprozent als Sprühgut Tröpfchen mit einem Sauterdurchmesser von ca. 8 µm, 10 µm und 15 µm bei einem Sprühbehälterdruck von 30 bar, 20 bar bzw 10 bar erzielt. Der Volumenstrom betrug dabei ca. 0,007 ml/s, 0,006 ml/s bzw. 0,004 ml/s. Rechnerisch kann der Inhalt eines Sprühbehälters mit einer Füllmenge von 100 ml so von knapp vier bis gut sieben Stunden Sprühdauer ausreichen.

## Patentansprüche

1. Sprühkopf (1) für eine Sprühdose,
welcher Behälterverbindungsmittel (4) zum Verbinden mit einem Sprühbehälter (2) aufweist, **dadurch gekennzeichnet,**
**dass** der Sprühkopf (1) mindestens zwei Austrittskanäle (15a, 15b) aufweist, welche so angeordnet sind, dass aus Austrittsöffnungen (16a, 16b) der Austrittskanäle (15a, 15b) austretende Sprühstrahle an einem von den Austrittsöffnungen (16a, 16b) beabstandeten Aufprallort zentral aufeinander treffen.

2. Sprühkopf (1) gemäß Anspruch 1, wobei zwischen jeweils zweien der Austrittskanäle (15a, 15b) der Abstand deren Eintrittsöffnungen (18a, 18b) größer ist als der Abstand deren Austrittsöffnungen (16a, 16b)

3. Sprühkopf (1) gemäß Anspruch 2, wobei die Austrittskanäle (15a, 15b) jeweils eine Mittelachse (17a, 17b) aufweisen, welche zur jeweiligen Mittelachse eines jeweils anderen der Austrittskanäle (15a, 15b) in einem Winkel (α) zwischen 30 und 120 Grad, vorzugsweise zwischen 45 und 90 Grad, steht.

4. Sprühkopf (1) gemäß einem der vorangehenden Ansprüche, wobei die vom Rand der jeweiligen Austrittsöffnungen (16a, 16b) aufgespannten Austrittsquerschnittsflächen zueinander geneigt sind.

5. Sprühkopf (1) gemäß einem der vorangehenden Ansprüche, wobei die Austrittskanäle (15a, 15b) jeweils einen kleinsten Durchmesser zwischen 5 und 50 µm, vorzugsweise zwischen 5 und 15 µm, aufweisen.

6. Sprühkopf (1) gemäß einem der vorangehenden Ansprüche, wobei die Austrittskanäle (15a, 15b) jeweils eine Länge von 50 bis 200 µm aufweisen.

7. Sprühkopf (1) gemäß einem der vorangehenden Ansprüche, wobei die Austrittskanäle (15a, 15b) jeweils ein als Quotient aus Länge des Austrittskanals und geringstem Durchmesser des Austrittskanals gebildetes Aspektverhältnis zwischen 3 und 25 aufweisen.

8. Sprühkopf (1) gemäß einem der vorangehenden Ansprüche, wobei die Austrittskanäle (15a, 15b) in einem gemeinsamen Düsenkörper (14), vorzugsweise einem Düsenkörper (14) aus Kunststoff, angeordnet sind.

9. Sprühkopf (1) gemäß einem der vorangehenden Ansprüche, wobei die Austrittskanäle (15a, 15b) mittels Laserbearbeitung hergestellt sind.

10. Sprühkopf (1) gemäß einem der vorangehenden Ansprüche, welcher mit einem Patientenluftwegeadapter integral geformt oder verbunden ist, der als in eine Patientennase einführbarer Nasenadapter, als einen Patientenmund und/oder eine Patientennase abdeckender Maskenadapter oder mit einem Patientenmund umschließbarer Adapter ausgebildet ist.

11. Sprühdose, welche einen Sprühkopf (1) gemäß einem der Ansprüche 1-10 und den Sprühbehälter (2) aufweist.

12. Sprühdose gemäß Anspruch 11, wobei der Druck im Sprühbehälter (2) 30 bar, vorzugsweise 15 bar nicht übersteigt.

13. Sprühdose gemäß einem der Ansprüche 11 und 12, wobei der Sprühbehälter (2) mit zumindest einem medizinischen Wirkstoff, vorzugsweise Sole, und einem Treibmittel befüllt ist.

14. Verfahren zum Herstellen eines Düsenkörpers (14) für einen Sprühkopf (1) gemäß einem der Ansprüche 1-13 wobei mittels Laserbearbeitung Düsenkörpermaterial zum Erzeugen eines Austrittskanals vom Düsenkörper (14) abgetragen wird.

15. Zerstäubungsverfahren,
**dadurch gekennzeichnet,**
**dass** Sprühgut mittels Behälterdrucks in Form mindestens zweier aufeinander prallender Sprühstrahle aus einem Sprühkopf (1) gedrückt wird.
